# EUROPEAN PATENT SPECIFICATION

(11) **EP 1 261 317 B1**
(45) Date of publication and mention of the grant of the patent: **10.10.2007**
(21) Application number: 01914710.7
(22) Date of filing: 07.03.2001
(51) Int. Cl.: A61K 9/10, A61P 27/02

(54) **METHODS AND COMPOSITIONS FOR TREATING AND PREVENTING POSTERIOR SEGMENT OPHTHALMIC DISORDERS**
VERFAHREN UND MITTEL ZUR BEHANDLUNG UND VORBEUGUNG VON ERKRANKUNGEN DER HINTEREN AUGENKAMMER
PROCEDES ET COMPOSITIONS POUR TRAITER ET PREVENIR DES AFFECTIONS OPHTALMIQUES DE LA PARTIE POSTERIEURE DE L'OEIL

(30) Priority: 10.03.2000 US 523102; 28.08.2000 US 648446
(43) Date of publication of application: 04.12.2002
(73) Proprietor: INSITE VISION INCORPORATED, Alameda, CA 94501 (US)
(72) Inventor: SI, Erwin, Chun-Chit, Alameda, CA 94502 (US); BOWMAN, Lyle, M., Pleasanton, CA 94502 (US); ROWE-RENDLEMAN, Cheryl, Walnut Creek, CA 94598 (US); ROY, Samir, San Ramon, CA (US)
(74) Representative: UEXKÜLL & STOLBERG
(86) International application number: PCT/US2001/007171
(87) International publication number: WO 2001/068053

(56) References cited:
- EP-A- 0 312 208
- EP-A- 1 040 837
- WO-A-00/07565
- WO-A-95/29696
- WO-A-96/03985
- WO-A-97/41844
- WO-A-98/10758
- WO-A-99/45929
- DATABASE BIOSIS [Online] BIOSCIENCES INFORMATION SERVICE, PHILADELPHIA, PA, US; 15 March 2000 (2000-03-15) COLOMBO S ET AL: "An eye drop form of an extracellular proteinase inhibitor prevents retinal neovascularization in an animal model." Database accession no. PREV200000251192 XP002183948 & IOVS, vol. 41, no. 4, 15 March 2000 (2000-03-15), page S640 Annual Meeting of the Association in Vision and Opthalmology.;Fort Lauderlade, Florida, USA; April 30-May 05, 2000
- DATABASE WPI Week 199919 Derwent Publications Ltd., London, GB; AN 1999-229406 XP002201344 & WO 99 13909 A (TOA GOSEI CHEM IND LTD)

### Sequence Listing

<110> Si, Erwin Chin-Chut
   Bowman, Lyle M.
   Rowe-Rendleman, Cheryl
   Roy, Samir
<120> Methods and Compositions for Treating and Preventing Posterior Segment Ophthalmic Disorders
<130> 13587.001
<150> US 09/523,102
   <151> 2000-03-10
<150> US 09/648,446
   <151> 2000-08-28
<160> 8
<210> 1
   <211> 11
   <212> PRT
   <213> Homo sapiens
<400> 1
   Met Arg Lys Pro Arg Cys Gly Asn Pro Asp Val
    1               5                  10
<210> 2
   <211> 12
   <212> PRT
   <213> Homo sapiens
<400> 2
   Met Arg Lys Pro Arg Cys GLy Val Pro Asp Val Gly
    1               5                  10
<210> 3
   <211> 3
   <212> PRT
   <213> Homo sapiens
<400> 3
   Arg Cys Gly
    1
<210> 4
   <211> 4
   <212> PRT
   <213> Homo sapiens
<400> 4
   Arg Cys Gly Val
    1
<210> 5
   <211> 5
   <212> PRT
   <213> Homo sapiens
<400> 5
   Arg Cys Gly Val Pro
    1               5
<210> 6
   <211> 6
   <212> PRT
   <213> Homo sapiens
<400> 6
   Arg Cys Gly Val Pro Asp
    1               5
<210> 7
   <211> 14
   <212> PRT
   <213> Artificial Sequence
<220>
   <223> Designed peptide based on matrix metalloproteinase conserved sequence
<400> 7
   Thr Met Arg Lys Pro Arg Cys Gly Asn Pro Asp Val Ala Asn
   1 5 10
<210> 8
   <211> 7
   <212> PRT
   <213> Artificial Sequence
<220>
   <223> Designed peptide based on vascular endothelial growth factor (VEGF)
   <223> sequence and capable of inhibiting VEGF binding to kinase domain receptor
<400> 8
   Ala Thr Trp Leu Pro Pro Arg
   1 5

## Description

### FIELD OF THE INVENTION

This invention relates to ophthalmic disorders, and more particularly to the prophylactic and therapeutic treatment of disorders associated with the posterior segment of the eye using topical ophthalmic compositions.

### BACKGROUND OF THE INVENTION

Posterior segment ophthalmic disorders affect the posterior segment of the eye, *i.e.,* the vitreous body, retina, optic nerve, and choroid. These disorders include such conditions as pathological neovascularization and ectopic proliferation, atrophy and nerve cell death, inflammation and infection, and detachment. Diseases and conditions commonly associated with these symptoms include macular degeneration, diabetic retinopathy, retinopathy of prematurity, retinitis pigmentosa, macular edema, glaucoma, posterior uveitis, endophthalmitis, ocular insult and ocular manifestation of systemic disease such as viral infection, arthritis and rosacea.

Atrophic and proliferative diseases of the vasculature and cells that support the posterior segment are primary causes of blindness in the world. Two vascular systems, retinal and uveal, support the posterior segment. The retinal vasculature is a complex of arteries and veins that supports the metabolic need of the neurons in the inner retina. With the exclusion of the fovea, which is a specialized region of photoreceptor cells in the center of the macula that is responsible for sharp color vision, the retina is comprised of 8 different cell types organized in 10 parallel layers. Light from the outside passes through the cornea, lens and vitreous and is focused on the macula, the region of highest visual acuity in the retina. Photoreceptors in the outer retina transduce light into neurochemical signals that are processed by retinal neurons and transmitted to the brain by the optic nerve. The uvea is a system of capillaries and arteries that lies just below the connective tissue exterior of the eye. The posterior part of the uvea, the choroid, is a layer of pigmented blood vessels sandwiched between the sclera and the retina. With the exception of the liver, the choroid has the richest blood supply of any vascular plexus, primarily to handle the metabolic needs of photoreceptors and the retinal pigment epithelium (RPE). The forward part of the uvea, the ciliary body, and the iris are more complex. However, infection in these anterior segment tissues is easily spread to the posterior segment by way of the uveal tract.

Neovascularization is a proliferative disease of endothelial cells which form the structural elements of blood vessels. Blood vessels grow and proliferate in a tissue, typically in response to a decrease in blood flow to the tissue. Neovascularization typically improves the flow of blood in a tissue by creating new blood vessels instead of replacing the existing vasculature. Pathological neovascularization involves both the degradation of tissue through enzymatic action and the formation of new immature blood vessels and fibrous membranes in ectopic locations. The formation of new blood vessels may compromise the function and complex architecture of arteries and veins in the uvea, retina, and choroid and ultimately cause the permanent loss of vision as a result of hemorrhaging and scarring. Trophic factors secreted by neovascular elements and adhesions produced from fibrous neovascular membranes further degrade visual function through detachment of the retina and the invasion of normally avascular regions with cytokines and inflammatory elements.

Neovascular diseases of the posterior segment of the eye include diabetic retinopathy, age-related macular degeneration (also known as wet or exudative macular degeneration), neovascular glaucoma, retinopathy of prematurity, sickle-cell retinopathy, retinal vein occlusion, oxygen induced retinopathy, and neovascularization due to ocular insults such as traumatic or surgical injury or transplantation of eye tissue. Other conditions or diseases associated with the manifestation of retinal neovascularization include any disease or condition where a part of the retina is subject to a relatively non-perfused state compared to surrounding tissue, where any one or more of the proteins, proteinases, hormones, or cellular signals associated with neovascularization are detected, or where new vessel growth can be detected or observed. In addition, diseases implicating matrix metalloproteinase (MMP) activity, endothelial cell invasion, or the generation of new blood vessels may also be associated with pathological neovascularization according to this invention.

Intraocular spaces in which inflammatory and immune responses can occur are the vitreous cavity and subretinal space. Constitutive expression of membrane-bound and soluble immunomodulatory factors and the induction of systemic immune deviation give the healthy eye a unique immunoprivileged microenvironment. However, when posterior segment ocular inflammation does occur, it is an important cause of blindness. According to this invention inflammatory diseases are those that display one of four distinct clinical features: variably sized chorio-retinal infiltrates of inflammatory cells; retinal vessel inflammation; vitreous cellular infiltrates; and edema of the macula, optic nerve head or entire retina. Other conditions include those where cells or cytokine profiles consistent with infection and inflammation can be detected. Inflammation is a special class of proliferative disease in which the proliferative elements are cells of the immune system. In these cases the tissue reacts to chemicals secreted by immune cells that are normally dormant or have been recruited to the site of inflammation. Inflammatory responses in the posterior segment may result from hypersensitivity (allergy), infectious agents, or unknown immunopathic cause. In hypersensitive inflammation, eosinophils are recruited. Upon degranulation these cells stimulate the release of a variety of mediators including granule proteins, prostaglandins, and cytokines that perpetuate the inflammatory response. When an infectious agent is the cause, the appropriate treatment is often straightforward, but appropriate drugs may have high side effect profiles.

Atrophic disorders of the posterior segment are diseases in which differentiated cells die and compromise vision. Cell death may be triggered by both environmental and genetic factors and may be distinguished as either apoptosis or necrosis. Atrophic cell death is not equivalent to normal programmed cell death that occurs during the developmental phase of the eye and its vasculature. For the purposes of this invention, atrophic diseases of the posterior segment include retinal atrophy that is triggered by trauma, systemic disease, vascular insufficiency, cellular senescence, or cytotoxic insult.

Age-related macular degeneration (ARMD) is one of the leading causes of blindness in older adults in the United States. The atrophic form of the disease (described below) accounts for 85% of the cases. The "wet" or exudative form is a neovascular disease that causes 90% of the blindness in Americans of European descent over the age of 65. Wet ARMD is characterized by loss of central vision, usually in both eyes, due to damage to the retinal pigment epithelial (RPE) cells and invasion of the avascular outer retina by immature blood vessels from the choroid capillaries. The invasion involves a breach of the RPE cell layer which constitutes the blood-retinal barrier. When invasion involves the macula, patients have serious visual impairment. The natural course of neovascularization in ARMD is the development of a disciform scar over the macula and irreversible blindness. In addition to their role in the regeneration of visual chromophore, 11-cis retinal, RPE cells partition the photoreceptor cells from the vascular layers of the choroid. The tissue invasion and degradation characteristic of neovascularization, and the loss of RPE and photoreceptor cells is facilitated by the action of proteinases, MMPs and the altered expression of integrins and integrin-binding elements. Alterations in the metabolism of invading blood vessels and invaded tissue (Bruch's membrane, RPE, and retina) also occur, and may result from a variety of events, such as the dysregulation of growth factors, the up-regulation of vascular endothelial growth factor (VEGF) and other molecules that signal proliferation, and the down-regulation of basic fibroblast growth factor (bFGF) and other signal molecules that regulate MMP activity. *See* Plantner JJ et al., Exp Eye Res. 67(6):637-45 (1998); Guo L et al., Invest Ophthalmol Vis Sci. 40(11):2676-82 (1999); Steen B et al., Invest Ophthalmol Vis Sci. 39(11):2194-200 (1998).

Diabetic retinopathy is the leading cause of blindness among working age adults in the United States. It is observed in up to ninety percent of patients with insulin dependent diabetes mellitus of long-term duration (more than 10 years). Klein et al., Arch. Ophth. 112:1217 (1994). Initially, the high blood glucose levels common to persons with diabetes mellitus cause an increase in glycosated proteins and growth factor levels in the eyes. This condition known as the "pre-diabetic retinopathy stage" and can lead to retinopathy if not prophylactically treated. Non-proliferative or early-stage diabetic retinopathy, also known as "background diabetic retinopathy," is characterized by thickening of the basement membrane, loss of retinal pericytes, microvascular abnormalities, intraretinal microaneurysms, retinal hemorrhages (known as "dot blot" or "cotton wool" spots), retinal edema, capillary closure, and soft and hard exudates. Late-stage or proliferative diabetic retinopathy is characterized by neovascularization and fibrovascular growth, i.e., scarring involving glial and fibrous elements, from the retina or optic nerve over the inner surface of the retina or into the vitreous cavity. At active neovascularization sites, both the high (54 kD) and low (33 kD) molecular weight forms of the protein urokinase have been found at levels significantly higher than in normal retinas. The levels of both pro and active forms of the matrix metalloproteinases MMP-2 (gelatinase A) and MMP-9 (gelatinase B) are also significantly elevated in neovascular membranes in comparison to normal membranes. *See* Das et al., Investigative Ophthalmology & Visual Sciences 40:809-13 (1999); Coors et al., Investigative Ophthalmology & Visual Sciences 40(4):S231 (1999). Typically the active forms of MMPs such as collagenase, stromelysin and gelatinase are not present at detectable levels in normal retinas.

Retinopathy of prematurity (ROP) is a common cause of blindness in children in the United States and the developed world. Premature infants are exposed to hyperoxic conditions after birth due to the higher partial pressure of oxygen in the atmosphere as compared to *in utero* conditions. Supplemental oxygen is necessary for the survival of premature infants, yet may result in ROP. The hyperoxic atmosphere causes retinal blood vessels to stop developing into the peripheral retina, resulting in ischemia and localized hypoxic conditions as the metabolic demands of the developing retina increase. The resulting localized hypoxia stimulates retinal neovascularization and fibrovascular growth into and above the retina and the vitreous. The neovascularization usually regresses, but may lead to irreversible vision loss. The therapeutic approach to ROP is prevention. *See* Gaynon MW and Stevenson DK, Pediatrics 105(2):295-310 (2000); Phelps DL et al., Cochrane Database Syst Rev. (2):CD001073 (2000). However, in severe cases laser diode, vitrectomy, or retinal ablation surgery may be unavoidable. There are at least 10,000 new cases per year of ROP with a worldwide estimate of 10 million total cases.

Glaucoma is a group of diseases characterized by a particular pattern of blindness involving damage to the optic nerve and visual field loss. Increased intra-ocular pressure is a risk factor and contributes to some but not all cases of optic nerve atrophy and retinal cell death in the glaucomatous eye. Race is a predisposing factor for severe glaucoma. People of African descent are six times more likely than those of European descent to develop bilateral blindness. Even when elevated intraocular pressure (IOP) is not exhibited, glaucomatous cell death occurs in the retina. Optic nerve damage occurs when the separations between axon bundles in the optic nerve and the retinal nerve fiber layer degenerates. Treatment for glaucoma usually focuses on lowering the IOP, which may alleviate the symptoms but cannot prevent all cell death. *See* Hitchings RA., Br J Ophthalmol. 84(7):678-9 (2000); Ritch R., Curr Opin Ophthalmol. 11 (2):78-84 (2000).

Atrophic cell death also occurs in "dry" or non-exudative age-related macular degeneration. Dry macular degeneration is the prevalent form of ARMD in patients over the age of 65. Unlike the neovascular form of the disease, race does not seem to be a risk factor in the development of dry ARMD. Early signs of the disease include a build up of material called drusen between the RPE and Bruch's membrane which impairs diffusion of oxygen from the choroidal circulation. Advanced disease is characterized by RPE and photoreceptor cell death often in localized regions of the peripheral retina (geographic atrophy), as well as alterations in the Bruch's membrane that prevents the proliferation and reattachment of RPE cells. Treatment for dry macular degeneration typically involves dietary supplementation with vitamin E and food supplements to enhance the metabolic capacity of the tissue and to restore the photoprotective pigments to the choroid and RPE. *See* Delcourt C et al., Arch Ophthalmol. 117(10):1384-90 (1999); Jacques PF, Int J Vitam Nutr Res. 69(3):198-205 (1999). Experimental surgical intervention to destroy drusen or transplant iris pigment epithelium or RPE cells may also be attempted. *See* Friberg TR, Semin Ophthalmol. 14(1):45-50 (1999); Abe T. et al., Tohoku J Exp Med. 189(4):295-305 (1999); Algverre PV et al., Eur J Ophthalmol. 9(3):217-30 (1999).

Retinitis pigmentosa (RP) is a group of inherited retinal diseases caused by mutations in photoreceptor or RPE proteins that affects 1 in 3000 individuals worldwide. The most common forms of RP involve mutations that alter the function or production of rhodopsin. RP is characterized by the selective loss of rod photoreceptors, and night blindness. Advanced disease includes constriction of the visual field (tunnel vision) and peripheral blindness. Patients exhibiting RP are generally blind by their third decade. Treatment for RP currently involves dietary supplementation with vitamin A palmitate. *See* Fex GA et al., Graefes Arch Clin Exp Ophthalmol. Aug:234 Suppl 1:S18-21 (1996). Chronic macular edema is a frequently observed complication in RP patients. Treatment with oral carbonic anhydrase inhibitors (e.g., acetazolamide) is commonly prescribed. See Wolfensberger, TJ Doc Ophthalmol. 97(3-4):387-97 (1999). Experimental gene therapy to replace photoreceptor proteins or enhance specific growth and survival factors has been successful in treating animal models of RP. *See* Ali RR et al., Nat Genet. Jul:25(3):306-10 (2000); Lewin AS et al., Nat Med. Aug;4(8):967-71 (1998).

Posterior uveitis is a group of sight-threatening inflammatory diseases of the back of the eye, including retinitis, retinochoroiditis, and choroiditis. Treatment for this condition generally involves systemic use of corticosteroid or other immuno-suppressive agents. However, systemic administration is not the ideal route of administration because of the potentially severe side effects.

Endophthalmitis is an inflammatory response to invading bacteria, or fungal and parasitic microorganisms. It generally involves the vitreous and retina and can lead to loss of sight within a short period of time, depending on the virulence of invading organisms and the degree of toxins produced by these organisms. It is a serious complication of cataract surgery, filtering procedures, and penetrating ocular injuries. The poor prognosis of the disease can be attributed mainly to the poor penetration of antibiotics into the back of the eye.

Known surgical and pharmacological treatments for posterior segment ophthalmic disease include surgical intervention such as vitrectomy, laser and radiotherapy, cryotherapy and pharmacological intervention such as chemotherapy. Panretinal laser coagulation is the classic treatment for proliferative diabetic retinopathy, but may have serious side effects such as foveal burns, hemorrhaging, retinal detachment, choroidal vessel growth, decreased peripheral and night vision, and changes in color perception. Vitrectomy, cryotherapy and laser therapy may be used to treat ROP and other neovascular diseases, but are not completely effective and may damage the eye and result in decreased vision. In some diseases such as exudative cases of age-related macular degeneration, temporary prevention of vision loss may be achieved by laser or photodynamic therapy, but no permanent treatment is available for some forms of diseases associated with retinal neovascularization.

Drug therapy has advantages compared to micro and laser surgery in that the tissue invasion is smaller and the stress placed on the ocular tissue is lower. The number of effective drugs, however, is quite small. In additional, patient compliance may be negatively influenced by systemic side effects. Compounds which inhibit the action of MMPs involved in connective tissue breakdown are of potential value in the treatment of angiogenesis-dependent diseases such as proliferative retinopathies, neovascular glaucoma, and other forms of retinal neovascularization. Certain agents have been proposed for inhibiting MMPs (*see* U.S. Patent No. 5,917,090). In particular, MMP inhibitors have been employed as potential treatments to the retina via intraorbital administration, tissue specific microinjection, or intravitreal injection (*see, e.g.,* European Patent Publication EP 0930067, published July 21, 1999; U.S. Patent No. 5,260,059; and published PCT Application WO 97/18835). While these treatments may act directly at the posterior segment, they have the disadvantage of being difficult to administer and of requiring the co-administration of anesthetic to the patient.

Topical treatment would be preferred because a topical composition may be self-administered by a patient, and does not require the co-administration of anesthetics. Topical compositions are generally ineffective at delivering a therapeutically effective amount of an active ingredient to the posterior segment, however, due to a lack of permeation through the conjunctiva, cornea, and sclera, and the presence of the blood-retinal barrier. Some ophthalmic treatment agents, such as the highly soluble β-blocking agents, have been found to reach the retina after topical administration because they are absorbed into the blood stream via the nasal mucosa or lid margin vessels and passed systemically to the retina (*see* Osborne et al., Exp. Eye Res. 69:331-42 (1999)). This method is ineffective for relatively insoluble agents that do not pass through the blood-retinal barrier and is undesirable if treatment of one eye is required. In addition, absorption into the blood stream may result in systemic side effects that may not result from low concentration topical administration and direct absorption by ocular tissues.

Compositions for topical treatment of a posterior segment ophthalmic disorder useful in preventing and treating any ocular neovascularization comprising one or more angiostatic compounds and a further suspension agent is described in the published PCT application WO 97/41844.

What is needed is a topical ophthalmic composition for the prophylactic and therapeutic treatment of posterior segment ophthalmic disorders that is capable of delivering a therapeutically effective amount of an active ingredient to the posterior segment. Also needed are methods for prophylactic and therapeutic treatments of posterior segment ophthalmic disorders.

### SUMMARY OF THE INVENTION

The present invention relates to the unexpected discovery that topical administration of the compositions of the present invention, which comprise a VEGF antagonist, are capable of delivering a therapeutically effective amount of a therapeutic agent to the vitreous body, retina, optic nerve aschoroid. This discovery was unexpected because prior methods of topical administration of relatively insoluble compounds failed to result in therapeutically effective amounts of the compound reaching the posterior segment tissue and being retained therein.

The present invention provides is the use of a VEGF antagonist for the preparation of a topical ophthalmic composition for use in a method for treating or preventing posterior segment ophthalmic disorders that affect the vitreous body, retina, optic nerve or choroid of a mammal in need of such treatment, the composition comprises a polymeric suspension agent and said VEGF antagonist, and wherein a topical administration of said composition delivers a therapeutically affective amount of said VEGF antagonist to said vitreous body, retina, optic nerve or choroid.

Further provided is the use of a VEGF antagonist for the preparation of a topical ophthalmic composition for use in a method for preventing ophthalmic disorders that affect the vitreous body, retina, optic nerve or choroid of mammal susceptible to developing such disorders, wherein said a composition comprises a polymeric suspension agent and said VEGF antagonist, and wherein topical administration of said composition delivers a therapeutically effective amount of said VEGF antagonist to said vitreous body, retina, optic nerve or choroid.

The present invention also provides topical ophthalmic compositions for use in treating or preventing posterior segment ophthalmic disorders that affect the vitreous body, retina, optic nerve or choroid of a mammal by topical administration to the eye said compositions comprising a therapeutically effective amount of a VEGF antagonist, and a polymeric suspension agent.

Further provided are topical ophthalmic compositions for use in treating or preventing posterior segment ophthalmic disorders that affect the vitreous body, retina, optic nerve or choroid of a mammal, where in said compositions are capable of delivering a therapeutically effective amount of the VEGF antagonist to the posterior segment of the eye.

The polymeric suspension agents used in the methods and compositions of the present invention may comprise one or more polymers. In particular, cross-linked polymers, acrylic acid-containing polymers and carboxyl-vinyl-containing polymers may be used. Particularly preferred polymers include polycarbophil, the DuraSite® polymeric delivery system (InSite Vision, Inc., Alameda, CA), and mucomimetic polymers (*see, e.g.,* U.S. Patent No. 5,932,572).

Additional advantages and features of the present invention will be apparent from the following detailed description, drawings and examples which illustrate preferred embodiments of the invention.

### BRIEF DESCRIPTION OF THE DRAWINGS

Figure 1 depicts the effects of a therapeutic agent on the number of neovascularization events in a section of murine retinal tissue;
Figure 2 depicts the relative levels of MMP-9 activity in control, hyperoxic untreated, and hyperoxic treated murine retinal tissue;
Figure 3 depicts the relative levels of MMP-2 activity in control, hyperoxic untreated, and hyperoxic treated murine retinal tissue;
Figure 4 depicts the relative levels of urokinase activity in control, hyperoxic untreated, and hyperoxic treated murine retinal tissue; and
Figure 5 depicts the retinal tissue levels of batimastat over time following topical application to rabbit eyes.

### DETAILLED DESCRIPTION OF PREFERRED EMBODIMENTS

Reference will now be made in detail to the presently preferred embodiments of the invention, which, together with the drawings and the following examples, serve to explain the principles of the invention. These embodiments are described in sufficient detail to enable those skilled in the art to practice the invention.

Unless otherwise defined, all technical and scientific terms used herein have the same meaning as commonly understood by one of ordinary skill in the art to which this invention belongs. Although any methods and materials similar or equivalent to those described herein can be used in the practice or testing of the present invention, the preferred methods, devices, and materials are now described

One skilled in the art may refer to general reference texts for detailed descriptions of known techniques discussed herein or equivalent techniques. These texts include Current Protocols in Molecular Biology (Ausubel et al., eds., John Wiley & Sons, N.Y., and supplements through June 1999), Current Protocols in Immunology (Coligan et al., eds., John Wiley & Sons, N.Y., and supplements through June 1999), Current Protocols in Pharmacology (Enna et al., eds., John Wiley & Sons, N.Y., and supplements through June 1999), The Pharmacological Basis of Therapeutics (Fingl et al., 1975), Remington's Pharmaceutical Sciences (Mack Publishing Co., Easton, PA, 18th edition (1990)), and Principles and Practice of Ophthalmology (Albert and Jakobiec eds., W.B. Saunders Company, (1994)) for example.

The present invention concerns uses and compositions for the prophylactic and therapeutic treatment of ophthalmic disorders, and particularly posterior segment ophthalmic disorders, with a therapeutic agent. The compositions and the methods employing them have been found to unexpectedly deliver therapeutically effective amounts of a VEGF antagonist to the posterior segment when administered topically to the eye of an animal. The therapeutic agent may be transported through the conjunctiva by active or facilitated transport, or by passive transport mechanisms.

The term VEGF antagonist refers to any pharmaceutically acceptable salt, derivative, stereoisomer, or mixture of stereoisomers of a VEGF antagonist, or to the VEGF antagonist itself. Pharmaceutically acceptable salts or derivatives of VEGF antagonists may also be used in the uses and compositions of the present invention. The term "pharmaceutically acceptable salt" used herein refers to those salts of the parent compound that do not significantly or adversely affect the pharmaceutical properties (*e.g*., toxicity, efficacy, etc.) of the parent compound. Pharmaceutically acceptable salts administrable by means of the compositions of this invention include, for example, chloride, iodide, bromide, hydrochloride, acetate, nitrate, stearate, palmoate, phosphate, and sulfate salts. Exemplary techniques for producing pharmaceutically acceptable derivatives include methylation, halogenation, acetylation, esterification, and hydroxylation. Other examples also include those selected or derived from those described in U.S. Patent No. 5,917,090.

VEGF antagonists includes compounds that antagonize receptors involved in posterior segment ophthalmic disorders, e.g., vascular endothelial growth factor (VEGF) receptors. VEGF antagonists include peptides that inhibit the binding of VEGF to its receptors, such as short disulfide-constrained peptides, and the synthetic peptide Ala-Thr-Trp-Leu-Pro-Pro-Arg (SEQ ID NO:8). *See* Fairbrother et al., Biochemistry 37(51):17754-64 (1998); Binetruy-Tournaire et al., EMBO J. 19(7):1525-33 (2000). VEGF antagonists inhibit the outgrowth of blood vessels by inhibiting the ability of VEGF to contact its receptors. This mechanism of anti-angiogenesis operates differently than the mechanism caused by the stimulation of growth factors such as bFGF, which act to inhibit angiogenesis by stimulating native inhibitors of proteases. Other VEGF antagonists may be derived from asymmetric variants of VEGF itself. *See, e.g.,* Siemester et al., Proceedings of the National Academy of Sciences U.S.A. 95:4625-29 (1998). Other useful VEGF antagonists are RNA aptamers, which may be designed to antagonize VEGF or the closely related platelet-derived growth factor (PDGF), and may be administered coupled to polyethylene glycol or lipids. *See, e.g.,* Floege et al., American Journal of Pathology 154(1):169-79 (1999); Ostendorf et al., J. Clin. Invest. 104(7):913-23 (1999); Willis et al., Bioconjug. Chem. 9(5):573-82 (1998).

In addition, the compositions of the invention may be enriched with one or more of the stereoisomers of a therapeutic agent, or may be substantially optically pure with respect to one stereoisomer. The methods for using therapeutic agents may also comprise enriched stereoisomers or mixtures. One skilled in the art is familiar with designing synthetic schemes that employ one or more optically pure reagents or intermediates, or stereoisomerically enriched reagents or intermediates, resulting in either a substantially optically pure composition or a stereoisomerically enriched composition. A substantially optically pure composition contains about 85 to about 95 percent, or higher, of one stereoisomer. Chromatographic, enzymatic, or selective crystallization techniques for enriching or purifying stereoisomers of therapeutic agents, mixtures of therapeutic agents, or any intermediate or reagent used to prepare a therapeutic agent may also be used.

In a preferred embodiment, the compositions of the present invention comprise a therapeutically effective amount of a VEGF antagonist. Preferred concentrations of VEGF antagonist(s) in the compositions of the present invention are in the range of about 0.01 to about 10 percent (wt/wt). A more preferred range of concentrations is from about 0.05 to about 5.0 percent, and even more preferred concentrations are from about 0.1 to about 1.0 percent.

As used herein, the term "therapeutically effective amount" means the total amount of each active component of the pharmaceutical composition or method that is sufficient to show a meaningful patient benefit, i.e., healing or amelioration of chronic conditions, a reduction in: neovascularization, inflammation, atrophy or other symptoms; an increase in rate of healing of such conditions, or a detectable change in the levels of MMP, cytokine profiles, or cellular activity or other related biochemical events in the retina or surrounding tissue. When applied to an individual active ingredient, administered alone, the term refers to that ingredient alone. When applied to a combination, the term refers to combined amounts of the active ingredients that result in the therapeutic effect, whether administered in combination, serially, or simultaneously.

Therapeutic efficacy and toxicity of the compositions may be determined by standard pharmaceutical, pharmacological, and toxicological procedures in cell cultures or experimental animals. For example, numerous methods of determining ED₅₀ (the dose therapeutically effective in 50 percent of the population) and LD₅₀ (the dose lethal for 50 percent of the population) exist. The dose ratio between therapeutic and toxic effects is the therapeutic index, and it can be expressed as the ratio ED₅₀/LD₅₀. Compositions exhibiting high therapeutic indices are preferred. The data obtained from cell culture assays or animal studies may be used in formulating a range of dosages for human use. The dosage is preferably within a range of concentrations that includes the ED₅₀ with little or no toxicity, and may vary within this range depending on the dosage form employed, sensitivity of the patient, and the route of administration.

The pH of the inventive compositions is preferably between about 5 and about 8, and may be adjusted for the particular therapeutic agent(s) used. Purified water USP and various acids and bases suitable for ophthalmic use, or combinations of acids and bases, may be used for adjusting the pH of the compositions. Non-limiting examples of acids and bases include acetic acid, boric acid, citric acid, lactic acid, phosphoric acid, hydrochloric acid, sodium hydroxide, sodium phosphate, sodium borate, sodium citrate, sodium acetate, sodium lactate, and TRIS.

The osmotic pressure of the compositions may be adjusted by methods known in the art to be between about 40 to about 400 milliosmolar (mOsM), more preferably between about 100 to about 300 mOsM. A preferred method of adjusting osmotic pressure is the addition of physiologically and ophthalmically acceptable salts. Sodium chloride, which approximates physiological fluid, is the preferred salt, for use in concentrations ranging from about 0.01 to about 1 percent by weight, or any value in that range. Preferably, the concentration is between about 0.1 to about 1 percent. Equivalent amounts of one or more salts made up of cations such as potassium, ammonium and the like and anions such as chloride, citrate, ascorbate, borate, phosphate, bicarbonate, sulfate, thiosulfate, bisulfite and the like, e.g., potassium chloride, sodium thiosulfate, sodium bisulfite, ammonium sulfate, and the like, can also be used in addition to or instead of sodium chloride to achieve osmotic pressures within the above-stated ranges.

Additional components of the composition may be chosen from any of those used in or capable of being used in a pharmaceutical formulation, especially those designed for topical administration to the eye. A non-exclusive list of components includes preservatives, stabilizers, chelating agents, dyes, antibiotics, antimicrobials, and anti-fungal agents. Preservatives such as benzalkonium chloride may be used in a range between about 0.001 to 1 percent by weight, or any value in this range. The compositions of the present invention may further comprise pharmaceutically acceptable carriers, excipients, gels, solutions, or diluents suitable for topical ophthalmic administration, and may include pharmaceutically acceptable polymeric suspension agents. Suitable carriers or excipients include, but are not limited to, calcium carbonate, calcium phosphate, various sugars, starches, cellulose derivatives, gelatin, and polymers such as polyethylene glycol. Suitable techniques for the formulation and administration of the compositions of the present invention may be found in Remington's Pharmaceutical Sciences, 18^{th} edition (1990).

In another preferred embodiment, the compositions of the present invention comprise a therapeutically effective amount of a VEGF antagonist and a pharmaceutically acceptable polymeric suspension agent that is capable of increasing the residence time of the VEGF antagonist in the eye. An increased residence time is desirable because it enables a greater amount of the VEGF antagonist to penetrate the conjunctiva or to otherwise be transported to the posterior segment of the eye, even at relatively low concentrations of VEGF antagonist. Generally, the concentration of VEGF antagonist is preferred to be kept relatively low in order to avoid systemic uptake and possible side effects.

Exemplary polymeric suspension agents include dextrans, polyethylene glycols, polyvinylpyrrolidone, polysaccharide gels, Gelrite®, cellulosic polymers like hydroxypropyl methylcellulose, and carboxyl-containing polymers such as polymers or copolymers of acrylic acid, as well as other polymeric demulcents. A preferred polymeric suspending agent is a water-swellable, water-insoluble polymer, especially a crosslinked carboxyl-containing polymer.

Crosslinked carboxyl-containing polymers that can be used in practicing this invention are, in general, well known in the art. In a preferred embodiment, these polymers may be prepared from at least about 90 percent, and preferably from about 95 to about 99.9 percent by weight, based on the total weight of monomers present, of one or more carboxyl-containing monoethylenically unsaturated monomers. Acrylic acid is the preferred carboxyl-containing monoethylenically unsaturated monomer, but other unsaturated, polymerizable carboxyl-containing monomers, such as methacrylic acid, ethacrylic acid, β-methylacrylic acid (crotonic acid), cis-α-methylcrotonic acid (angelic acid), trans-α-methylcrotonic acid (tiglic acid), α-butylcrotonic acid, α-phenylacrylic acid, α-benzylacrylic acid, α-cyclohexylacrylic acid, β-phenylacrylic acid (cinnamic acid), coumaric acid (o-hydroxycinnamic acid), umbellic acid (p-hydroxycoumaric acid), and the like may be used in addition to or instead of acrylic acid.

The polymers may be crosslinked by a polyfunctional crosslinking agent, preferably a difunctional crosslinking agent. The amount of crosslinking should be sufficient to form insoluble polymer particles, but not so great as to unduly interfere with sustained release of the therapeutic compound. Typically the polymers are only lightly crosslinked. Preferably the crosslinking agent is contained in an amount of from about 0.01 to about 5 percent, preferably from about 0.1 to about 5.0 percent, and more preferably from about 0.2 to about 1 percent, based on the total weight of monomers present.

Suitable crosslinking agents include non-polyalkenyl polyether difunctional crosslinking monomers, such as divinyl glycol; 2,3-dihydroxyhexa-1,5-diene; 2,5-dimethyl-1,5-hexadiene; divinylbenzene; N,N-diallylacrylamide; N,N-diallylmethacrylamide, and the like. Other suitable crosslinking agents include polyalkenyl polyether crosslinking agents such as polyallyl sucrose or polyallyl pentaerythritol (*see*, *e.g.,* U.S. Patent No. 2,798,053), and diolefinic non-hydrophilic macromeric crosslinking agents as disclosed in U.S. Patent Nos. 4,192,827 and 4,136,250.

The crosslinked carboxyl-vinyl polymers may be made from a carboxyl-vinyl monomer or monomers as the sole monoethylenically unsaturated monomer present, together with a crosslinking agent or agents. Preferably, the polymers are ones in which up to about 40 percent, and preferably from about 0 to about 20 percent by weight, of the carboxyl-containing monoethylenically unsaturated monomer or monomers has been replaced by one or more non-carboxyl-containing monoethylenically unsaturated monomer or monomers containing only physiologically and ophthalmically innocuous substituents, including acrylic and methacrylic acid esters such as methyl methacrylate, ethyl acrylate, butyl acrylate, 2-ethylhexylacrylate, octyl methacrylate, 2-hydroxyethyl-methacrylate, 3-hydroxypropylacrylate, and the like, vinyl acetate, N-vinylpyrrolidone, as well as the monoethylenically unsaturated monomers disclosed in U.S. Patent No. 4,548,990.

Particularly preferred polymers are lightly crosslinked acrylic acid polymers wherein the crosslinking monomer is 2,3-dihydroxyhexa-1,5-diene or 2,3-dimethylhexa-1,5-diene. Preferred commercially available polymers include polycarbophil (Noveon AA-1) and Carbopol®. Most preferably, the polycarbophil-containing DuraSite® polymeric delivery system (InSite Vision, Inc., Alameda, CA), which is a sustained release topical ophthalmic delivery system that releases a drug at a controlled rate, is used as the polymeric suspension agent in the compositions of the present invention.

The crosslinked carboxyl-vinyl polymers used in practicing this invention are preferably prepared by suspension or emulsion polymerizing the monomers, using conventional free radical polymerization catalysts, to a dry particle size of not more than about 1 to 10 µm in equivalent spherical diameter; e.g., to provide dry polymer particles ranging in size from about 1 to about 30 µm, and preferably from about 5 to about 20 µm, in equivalent spherical diameter. Using polymer particles that were obtained by mechanically milling larger polymer particles to this size is preferably avoided. In general, such polymers will have a molecular weight that has been variously reported as being from about 250,000 to about 5,000,000,000.

In the most preferred embodiment of the invention, the particles of crosslinked carboxyl-vinyl polymer are monodisperse, meaning that they have a particle size distribution such that at least 80 percent of the particles fall within a 10 µm band of major particle size distribution. More preferably, at least 90 percent and most preferably at least 95 percent, of the particles fall within a 10 µm band of major particle size distribution. Also, a monodisperse particle size means that there is no more than 20 percent, preferably no more than 10 percent, and most preferably no more than 5 percent particles of a size below 1 µm. The use of a monodispersion of particles will give maximum viscosity and an increased eye residence time of the ophthalmic medicament delivery system for a given particle size. Monodisperse particles having a particle size of 30 µm and below are most preferred. Good particle packing is aided by a narrow particle size distribution.

The compositions of the present invention normally contain 0.01 to 10 percent, preferably 0.05 to 5.0 percent, more preferably 0.1 to 1.03 percent, of the therapeutic agent, and 0.1 to 10 percent, preferably 0.5 to 6.5 percent, of a polymeric suspension agent. In the case of the above described water-insoluble, water-swellable crosslinked carboxyl-vinyl polymer, a more preferred amount of the polymeric suspending agent is an amount ranging from about 0.5 to about 2.0 percent, or any chosen range between these percentages. Especially preferred embodiments comprise from about 0.5 percent to about 1.3 percent polymer, and in certain embodiments from about 0.6 to about 0.9 percent, based on the weight of the composition. Although referred to in the singular, it should be understood that one or more species of polymeric suspension agent, such as the crosslinked carboxyl-containing polymer, may be used with the total amount falling within the stated ranges. In one preferred embodiment, the composition contains from about 0.5 to about 2.0 percent, or any chosen range between these percentages, of a polycarbophil, such as NOVEON AA-1, and even more preferred is from about 0.60 to about 1.3 percent.

In one embodiment, the amount of insoluble lightly crosslinked carboxyl-vinyl polymer particles, the pH, and the osmotic pressure can be correlated with each other and with the degree of crosslinking to give a composition having a viscosity in the range of from about 500 to about 100,000 centipoise, and preferably from about 1,000 to about 30,000 or about 1,000 to about 10,000 centipoise, as measured at room temperature (about 25° C) using a Brookfield Digital LVT Viscometer equipped with a number 25 spindle and a 13R small sample adapter at 12 rpm. Alternatively, when the viscosity is within the range of 500 to 3000 centipoise, it may be determined by a Brookfield Model DV-11+, choosing a number cp-52 spindle at 6 rpm. One skilled in the art is familiar with methods for adjusting and optimizing viscosity ranges for pharmaceutical compositions. When water soluble polymers such as hydroxypropyl methylcellulose (HPMC) are used as suspension agents, the viscosity will typically be about 10 to about 400 centipoise, more typically about 10 to about 200 centipoises or about 10 to about 25 centipoise.

Also provided are methods for prophylactic and therapeutic treatment of diseases and conditions manifesting or associated with the posterior segment of the eye comprising administering a topical ophthalmic composition of the present invention to an animal in need of such treatment.

The duration of prophylactic and therapeutic treatment will vary depending on the particular disease or condition being treated. Some diseases lend themselves to acute treatment whereas others require long-term therapy. For example, proliferative retinopathy can reach a threshold in a matter of days as seen in ROP, some cases of diabetic retinopathy, and neovascular glaucoma. Premature infants are at risk for neovascularization around what would be 35 weeks gestation, a few weeks after birth, and will remain at risk for a short period of time until the retina becomes vascularized. Diabetic retinopathy can be acute but may also remain in the proliferative phase for a longer period of time. Diabetic retinopathy will eventually become quiescent as the vasoproliferative signal diminishes due to neovascularization and destruction of the retina.

The products and processes of the present invention are illustrated by the following examples, which do not fall within the scope of the present claims.

### EXAMPLE 1

### Preparation of Compositions Containing a Therapeutic Agent

Various formulations of the compositions of the present invention were compounded, and are listed in the following tables.

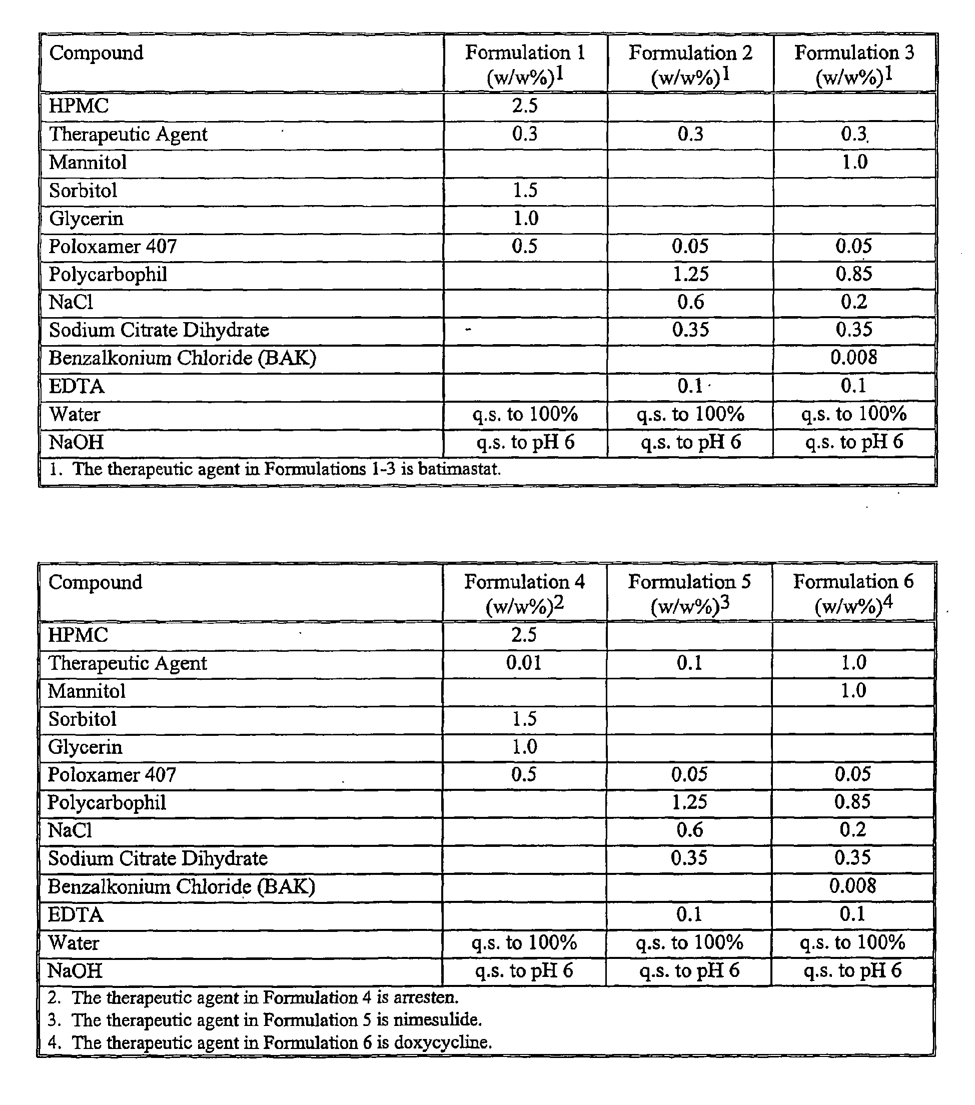

The physical and chemical characteristics of the compositions of the invention may be modified or optimized according to the skill in the art. Thus, pH, osmotic pressure, viscosity, and the content of various additional components may be chosen from any appropriate range known or modified from the examples given here. The methods for preparing and selecting exemplary formulations containing polymeric suspension agents are described in U.S. Patent Nos. 5,188,826 and 5,192,535, for example. In general, the pharmaceutical compositions of the invention may be manufactured in a manner that is itself known, *e.g*., by means of conventional mixing, dissolving, emulsifying, encapsulating, entrapping, or lyophilizing processes.

The compositions may be formulated in any of several ways. For example, the lightly-crosslinked polymer particles, the therapeutic agent, the osmotic pressure-adjusting salt and any of the optional ingredients also being used may be pre-blended, added to all or part of the water, and stirred vigorously until apparent polymer dispersion is complete, as evidenced by the absence of visible polymer aggregates, which usually occurs within about an hour. Sufficient pH adjusting agent is then added incrementally to reach the desired pH, and more water to reach 100 percent formula weight can be added at this time, if necessary.

Another convenient method involves adding the therapeutic agent to about 95 percent of the final water volume and stirring for a sufficient time to saturate the solution. Solution saturation can be determined in a known manner, *e.g*., using a spectrophotometer. The lightly crosslinked polymer particles and the osmotic pressure-adjusting salt are first blended in dry form and then added to the drug-saturated suspension and stirred until apparent polymer hydration is complete. Following the incremental addition of sufficient pH adjusting agent to reach the desired pH, the remained of the water is added, with stirring, to bring the composition to 100 percent formula weight.

A preferred method of formulating the topical ophthalmic compositions of this invention involves adding the polymer to 90 grams of water per 100 grams of gel, then stirring for about 1 hour until the gel is fully hydrated. The therapeutic agent is then added as an aqueous solution or a suspension, with stirring. Next, sodium chloride is added as a solid, together with sufficient water to bring the mass to 100 grams, and the pH is adjusted to the final pH, *e.g*., with 10N sodium hydroxide.

The prepared composition is then sterilized, preferably by briefly heating, e.g., for about 30 minutes with steam at about 121 degrees Celsius, and then filled into appropriate containers. Alternatively, the formulation may be sterilized by dissolving the drug in an appropriate solvent, sterile filtering and precipitating the drug in the formulation. Preservative-free compositions may be filled into unit-dose containers, at the preferred viscosity, eliminating the potential for preservative-related irritation and sensitization of the corneal epithelium, as has been observed to occur particularly from ophthalmic medicaments containing preservatives such as mercurial preservatives.

Compositions containing preservatives may also be filled into multiple-dose containers at the preferred viscosity, if desired, particularly because the viscosities of the compositions of this invention permit constant, accurate dosages to be administered dropwise to the eye as many times each day as necessary. In those compositions where preservatives are to be included, suitable preservatives include benzalkonium chloride in amounts ranging from about 0.001 to about 0.02 percent, chlorobutanol, preferably at about 0.5 percent, chlorobutanol chloral derivative, preferably at about 0.5 percent, methyl paraben and propyl paraben, preferably about 0.01 to about 0.05 percent, sorbic acid, preferably about 0.2 percent, Cetrimide, preferably about 0.01 percent, polyquat, preferably about 0.001 percent, cetyl bromide, preferably about 0.01 percent, and the like, each of the foregoing preservative amounts being based on the total weight of the composition.

### EXAMPLE 2

### Topical Ophthalmic Administration of a Composition Containing a Therapeutic Agent in Mice

A newborn mouse animal model of retinopathy of prematurity, which may be used as a model of retinal neovascularization conditions and diseases such as diabetic retinopathy and oxygen-induced blindness, demonstrates the utility and surprisingly advantageous topical ophthalmic administration of a therapeutic agent to the posterior segment of the eye.

Newborn mice on postnatal day 7 are kept in high oxygen (75 percent) from day 7 to 11, and then are brought to normal room air on day 12. A relative hyperoxia results, and retinal neovascularization is seen in 100 percent of the exposed animals by day 17. Some of the animals (n = 9) initially exposed to the oxygen cycle receive Formulation 1 eyedrops four times a day in the right eye on days 14-17 (four days) (Group I). Some animals (n = 9) receive the same eyedrops, administered similarly, on days 13-17. In another group of animals (n = 9) (Group III), the eyedrops are instilled in the right eye and normal saline is instilled in the left eye (control). Animals are sacrificed on day 17. Newborn animals kept in room air only for 17 days may serve as controls.

Eye tissue is processed for paraffin embedding sections, which are stained for nuclei with DAPI (diamidinophenylindole). Sections can be examined under a fluorescence microscope and nuclei on the vitreous side of the inner limiting membrane of the retina, representing microvascular cells, are counted in each section using a masked protocol.

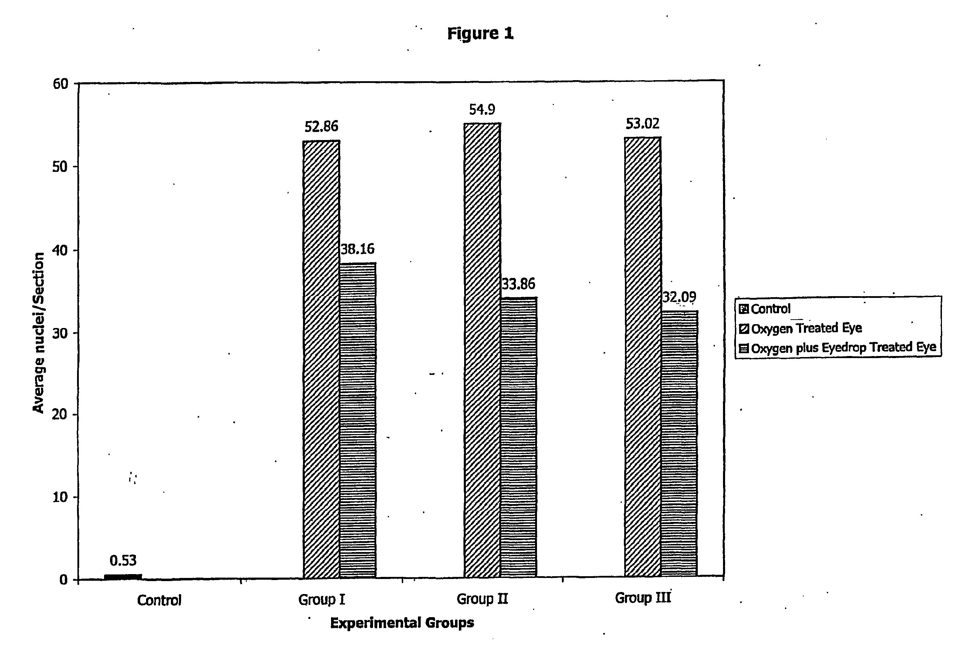

Numerous neovascular tufts may be seen protruding from the retina into the vitreous in animals exposed to hyperoxia (75 percent oxygen) followed by room air. Quantification reveals about 52.86 neovascular nuclei per section in experimental animals (without drug treatment) compared to controls (0.53 neovascular nuclei per section). Animals treated with Formulation 1 eyedrops show a decrease in neovascular nuclei by 27.8 percent (Group I), 38.3 percent (Group II) and 39.47 percent (Group III) respectively (Fig. 1).

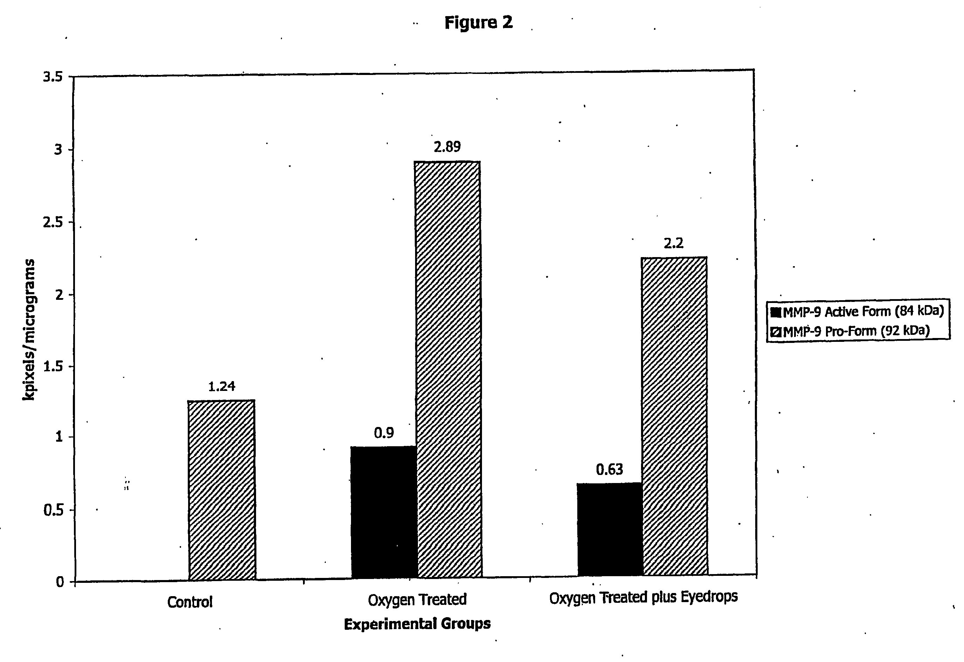

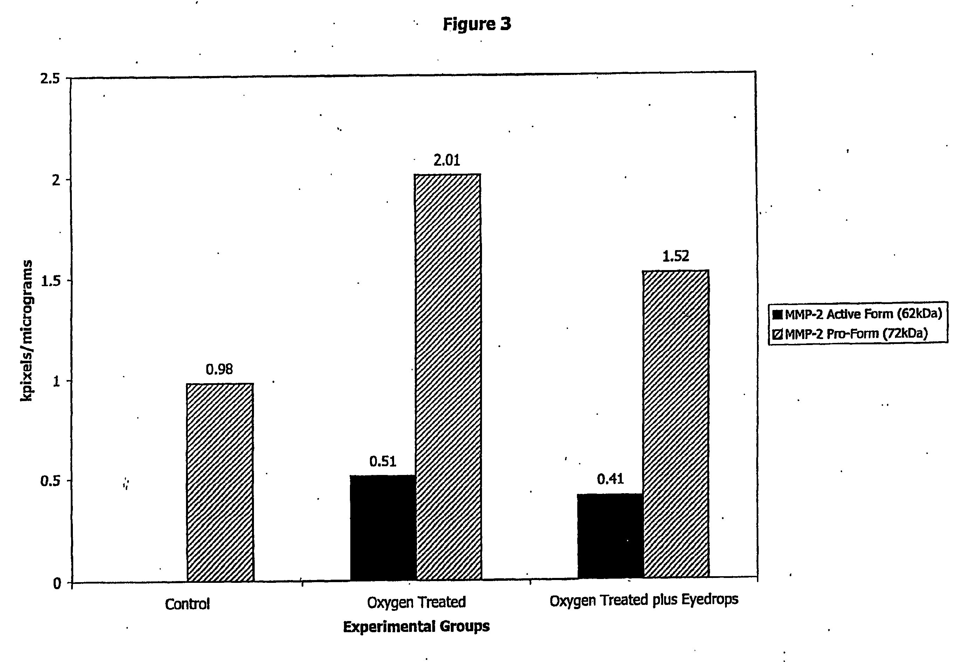

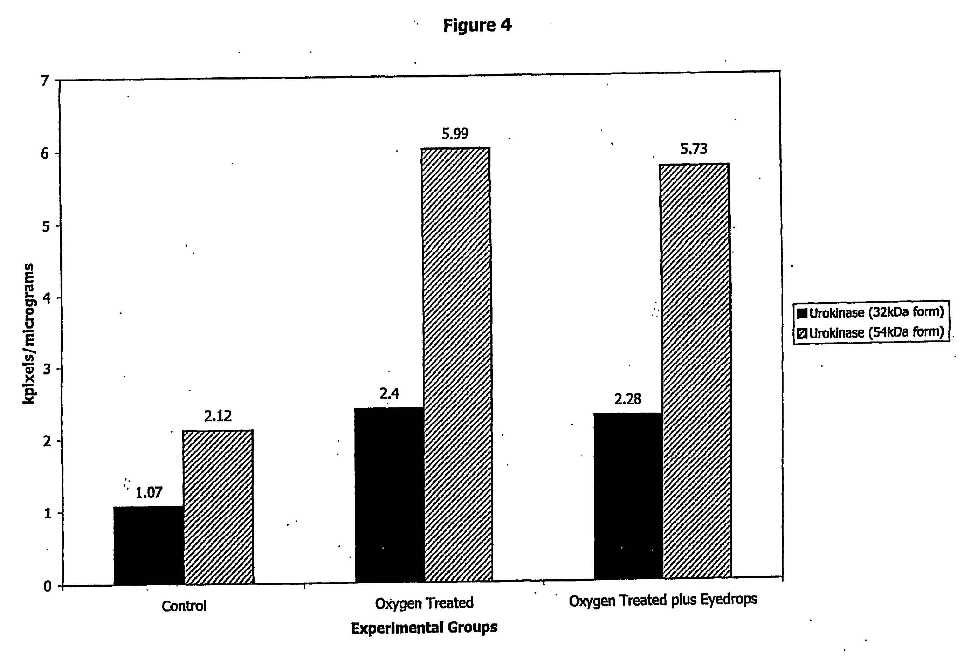

Analysis of MMP-2 activity in animals with retinal neovascularization shows a significant increase in the pro-form of MMP-2 (72 kDa) compared to controls (Fig. 2). The active form of MMP-2 (62 kDa), which is undetectable in controls, is also significantly increased in animals with neovascularization. The Formulation 1 eyedrops decrease the activity of both pro- and active forms of MMP-2. Analysis of MMP-9 activity in animals with retinal neovascularization shows a significant increase in the pro-form of MMP-9 (92 kDa) compared to controls (Fig. 3). The active form of MMP-9 (84 kDa), which is undetectable in controls, is significantly increased in animals with retinal neovascularization. Treatment with Formulation 1 eyedrops decreases the activity of both pro- and active forms of MMP-9 significantly. Animals with retinal neovascularization also show a significant increase in both the 32 kD and 54 kD molecular weight forms of urokinase compared to controls. Zymographic analysis does not show any change in activity of either form of urokinase in animals treated with Formulation 1 eyedrops (Fig. 4).

In another study, animals are treated with hyperoxia conditions, as described above. Then, batimastat is introduced into the animals via intraperitoneal injection (IP). A course of IP injection on days 12, 14 and 16 results in a 72 percent reduction in neovascularization in the same animal model (Das et al., Archives of Ophthalmology, 117:498-503 (1999)).

The result of Formulation 1 eyedrop formulation on the inhibition of retinal neovascularization is significant and the effect is specific to MMP inhibition, as shown by zymographic analysis and urokinase assays (Fig. 4). These results also show that the drug effectively reaches the retinal tissue and, thus, can be used to treat the retina via topical delivery.

### EXAMPLE 3

### Topical Ophthalmic Administration of a Composition Containing a Therapeutic Agent in Rabbits

A study in rabbits will examine the ¹⁴C activity in ocular tissues and blood plasma following administration of a topical ophthalmic composition containing 0.3 percent labeled therapeutic agent (batimastat) in the DuraSite® delivery vehicle. The ocular tissues to be examined are the aqueous humor, cornea, iris and ciliary body, vitreous humor, retina and choroid, and the sclera. The study will proceed in three step-wise phases: phase 1 will examine ¹⁴C activity at 20 minutes, 40 minutes, 1 hour and 2 hours post-administration, phase 2 will examine ¹⁴C activity at 3 and 4 hours post-administration, and phase 3 will examine ¹⁴C activity at 6 and 8 hours post-administration. Initially, 6 rabbits (12 eyes) will be examined at each time point. If any phase of the study is not executed, the rabbits allocated to that phase may be reassigned to other time points to better estimate bioavailability and pharmacokinetics. Nor more than 12 rabbits will be assigned to any single time point. It is anticipated that 48-54 total rabbits will be used in the study. To control bias, animals will be randomly enrolled in the study. Within an animal room (total capacity 54 rabbits), each rabbit eligible for enrollment in the study will be randomly assigned a temporary sequential number. Rabbits will then be selected for use in the study in sequential order of temporary number.

This study will use 48-54 female New Zealand White rabbits that weigh approximately 1.8 to 2.8 kg upon arrival and will be approximately 9 weeks old. Each rabbit will be identified with an ear tag bearing a unique number, and the rabbit's cage will also bear the same number. Rabbits will be acclimated to the laboratory environment in a specified quarantine area for a minimum of two weeks before being used in the study. Rabbits will receive a daily ration of commercially available feed and tap water *ad libitum.* Rabbit health will be monitored daily. Rabbits will be placed in temporary housing after instillation of ¹⁴C-labeled test material. The rabbits will be anesthetized and euthanized at the conclusion of the *in vivo* experimental period.

The test material will be administered with a positive displacement micropipettor. The average mass and standard deviation of dispensed test material will be estimated. Approximately 25 mg of test material will be instilled into the lower cul-de-sac of both eyes. The material will be placed into the eye by gently pulling the lower lid away from the globe to form a cup into which the material will be instilled.

Rabbits will be anesthetized with an intramuscular injection of ketamine and xylazine (0.4ml/kg each) approximately 20 minutes prior to the scheduled collection time of aqueous humor. Aqueous humor will be collected from both eyes according to methods known in the art. Aqueous humor will be collected from OD, then OS. A 0.5 ml syringe with a fixed 28G x ½" needle will be used, and an ophthalmic solution of 0.5 percent proparacaine hydrochloride will be administered prophylactically to all eyes without testing for corneal reflex. Eyes will be irrigated with commercially available Eye Irrigating Solution. Both eyes will be enucleated starting with OD. Tissues collected will include: bulbar conjunctiva, cornea, iris, sclera, vitreous humor, and retina. Tissues will be placed into preweighed scintillation vials. All scintillation vials will be capped and weighed. Immediately after the aqueous humor has been collected from both eyes, approximately 5 ml of blood will be withdrawn by intracardiac puncture. Blood will be collected into a heparinized tube.

For the bulbar conjunctiva, cornea, iris, sclera and retina/choroid, 100 µl of reverse osmosis (RO) purified water will be added to each sample and the sample vortexed. 250 µl of hyamine hydroxide will be added to each vial and the sample again vortexed. The samples will then be incubated at 55 degrees Celsius in a water bath until solubilized (approximately 1-4 hours). After solubilization is complete, the samples will again be vortexed, and 6 ml of CytoScintES® scintillation cocktail will be added to each sample. Solubilized samples will be immediately mixed with the scintillation cocktail by repeatedly inverting the capped vial. Vigorous shaking will be avoided.

For the vitreous humor, 100 µl of reverse osmosis (RO) purified water will be added to each sample and the sample vortexed. 750 µl of hyamine hydroxide will be added to each vial and the sample again vortexed. The samples will then be incubated at 55 degrees Celsius in a water bath until solubilized. After solubilization is complete, the samples will again be vortexed, and approximately 18 ml of CytoScintES® scintillation cocktail will be added to each sample. Solubilized samples will be immediately mixed with the scintillation cocktail by repeatedly inverting the capped vial. Vigorous shaking will be avoided.

For the aqueous humor, 6 ml of CytoScintES® scintillation cocktail will be added directly to each aqueous humor sample and mixed by repeatedly inverting the capped vial. There is not need to solubilize the aqueous humor sample. Blood samples will be kept in a cooler containing ice packs to keep them cold until separation. Plasma will be separated from the red blood cells by centrifugation at 4 degrees Celsius and 1200-1500g for 15 minutes. Immediately after centrifuging, 1 ml of the plasma will be pipetted from the tube and placed into a 20 ml scintillation vial. 18 ml of CytoScintES® scintillation cocktail will be added directly to each plasma sample and mixed by repeatedly inverting the capped vial. All samples will be dark-adapted overnight before counting in a Beckman LS 3801.

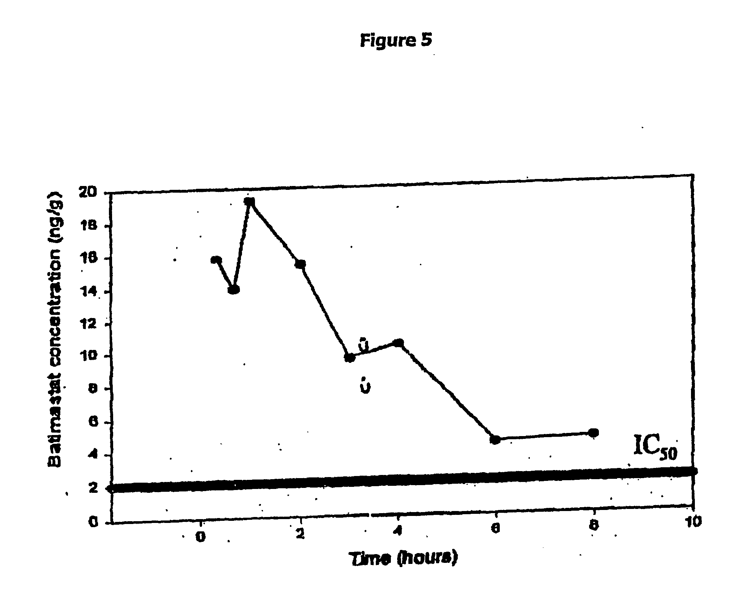

The topical batimastat composition reaches the retinal tissue shortly after topical administration to the eye, and is maintained in the retinal tissue at therapeutically effective levels for at least 8 hours, as shown in Fig. 5. These results show that the drug effectively reaches the retinal tissue and thus can be used to treat the retina via topical delivery.

## Claims

1. The use of a VEGF antagonist for the preparation of a topical ophthalmic composition for use in a method for treating or preventing posterior segment ophthalmic disorders that affect the vitreous body, retina, optic nerve or choroid of a mammal in need of such treatment, wherein said composition comprises a polymeric suspending agent and said VEGF antagonist, and wherein topical administration of said composition delivers a therapeutically effective amount of said VEGF antagonist to said vitreous body, retina, optic nerve or choroid.

2. The use according to claim 1, wherein the mammal is a human.

3. The use according to claim 1, wherein the polymeric suspension agent comprises polycarbophil.

4. The use according to claim 3, wherein the polycarbophil is present at a concentration of about 0.5 to about 1.5 percent by weight.

5. The use according to claim 1, wherein the VEGF antagonist is an RNA aptamer designed to antagonize VEGF.

6. The use according to claim 1, wherein the VEGF antagonist is a neutralizing antibody that targets a VEGF receptor.

7. The use according to claim 1, wherein the VEGF antagonist is a peptide that inhibits the binding of VEGF to a VEGF receptor, or an asymmetric variant of VEGF.

8. The use according to claim 7, wherein said VEGF antagonist is a peptide that inhibits the binding of VEGF to a VEGF receptor.

9. The use according to claims 7 or 8, wherein said peptide is a short disulfide-constrained peptide.

10. The use according to claim 9, wherein said peptide has the sequence Ala-Thr-Trp-Leu-Pro-Pro-Arg (SEQ ID NO:8).

11. The use according to claim 1, wherein said composition consists essentially of a said polymeric suspension agent and said VEGF antagonist.

12. The use of a VEGF antagonist for the preparation of a topical ophthalmic composition for use in a method for preventing posterior segment ophthalmic disorders that affect the vitreous body, retina, optic nerve or choroid of a mammal susceptible to developing such disorders, wherein said composition comprises a polymeric suspending agent and said VEGF antagonist, and wherein topical administration of said composition delivers a therapeutically effective amount of said VEGF antagonist to said vitreous body, retina, optic nerve or choroid.

13. An topical ophthalmic composition for use in treating or preventing posterior segment ophthalmic disorders that affect the vitreous body, retina, optic nerve or choroid of a mammal by topical administration to the eye, said composition comprising a therapeutically effective amount of a VEGF antagonist and a polymeric suspension agent.

14. The composition of claim 13, wherein the polymeric suspension agent comprises polycarbophil.

15. The composition of claim 14, wherein the polycarbophil is present at a concentration of about 0.5 to about 1.5 percent by weight.

16. The composition of claim 13, wherein the VEGF antagonist agent is an RNA aptamer designed to antagonize VEGF.

17. The composition of claim 13, wherein the VEGF antagonist is a neutralizing antibody that targets a VEGF receptor.

18. The composition of claim 13, wherein said composition is capable of delivering a therapeutically effective amount of the VEGF antagonist to the posterior segment of the eye.

19. The composition according to claim 13, wherein said composition consists essentially of a said polymeric suspension agent and said VEGF antagonist.

## Patentansprüche

1. Verwendung eines VEGF-Antagonisten für die Herstellung einer topischen, ophthalmischen Zusammensetzung für die Verwendung in einem Verfahren zur Behandlung oder zur Vorbeugung ophthalmischer Erkrankungen im hinteren Abschnitt, die den Glaskörper, die Retina, den Sehnerv oder die Aderhaut eines Säugetieres betreffen, das eine derartige Behandlung benötigt, wobei die Zusammensetzung ein polymeres Suspensionsmittel und den VEGF-Antagonisten umfasst, und wobei die topische Verabreichung der Zusammensetzung dem Glaskörper, der Retina, dem Sehnerv oder der Aderhaut eine therapeutisch wirksame Menge des VEGF-Antagonisten bereitstellt.

2. Verwendung gemäß Anspruch 1, bei der das Säugetier ein Mensch ist.

3. Verwendung gemäß Anspruch 1, bei der das polymere Suspensionsmittel Polycarbophil umfasst.

4. Verwendung gemäß Anspruch 3, bei der das Polycarbophil in einer Konzentration von ungefähr 0,5 bis ungefähr 1,5 Gew.-% vorliegt.

5. Verwendung gemäß Anspruch 1, bei welcher der VEGF-Antagonist ein RNA-Aptamer ist, der so gestaltet ist, dass er VEGF antagonisiert.

6. Verwendung gemäß Anspruch 1, bei welcher der VEGF-Antagonist ein neutralisierender Antikörper ist, der auf einen VEGF-Rezeptor gerichtet ist.

7. Verwendung gemäß Anspruch 1, bei welcher der VEGF-Antagonist ein Peptid, das die Bindung von VEGF an einen VEGF-Rezeptor inhibiert, oder eine asymmetrische Variante des VEGF ist.

8. Verwendung gemäß Anspruch 7, bei welcher der VEGF-Antagonist ein Peptid ist, das die Bindung von VEGF an einen VEGF-Rezeptor inhibiert.

9. Verwendung gemäß Anspruch 7 oder 8, bei der das Peptid ein kurzes, über Disulfid eingeschränktes Peptid ist.

10. Verwendung gemäß Anspruch 9, bei der das Peptid die Sequenz Ala-Thr-Trp-Leu-Pro-Pro-Arg (SEQ ID NO: 8) aufweist.

11. Verwendung gemäß Anspruch 1, bei der die Zusammensetzung im Wesentlichen aus dem polymeren Suspensionsmittel und dem VEGF-Antagonisten besteht.

12. Verwendung eines VEGF-Antagonisten für die Herstellung einer topischen, ophthalmischen Zusammensetzung für die Verwendung in einem Verfahren zur Verbeugung ophthalmischer Erkrankungen im hinteren Abschnitt, die den Glaskörper, die Retina, den Sehnerv oder die Aderhaut eines Säugetiers betreffen, das anfällig für der Ausbildung derartiger Erkrankungen ist, wobei die Zusammensetzung ein polymeres Suspensionsmittel und den VEGF-Antagonisten umfasst, und wobei die topische Verabreichung der Zusammensetzung dem Glaskörper, der Retina, dem Sehnerv oder der Aderhaut eine therapeutisch wirksame Menge des VEGF-Antagonisten bereitstellt.

13. Topische, ophthalmische Zusammensetzung zur Verwendung bei der Behandlung oder der Vorbeugung ophthalmischer Erkrankungen des hinteren Abschnitts, die den Glaskörper, die Retina, den Sehnerv oder die Aderhaut eines Säugetiers betreffen, durch topische Verabreichung der Zusammensetzung, die eine therapeutisch wirksame Menge eines VEGF-Antagonisten und eines polymeren Suspensionsmittels umfasst, in das Auge.

14. Zusammensetzung gemäß Anspruch 13, wobei das polymere Suspensionsmittel Polycarbophil umfasst.

15. Zusammensetzung gemäß Anspruch 14, wobei das Polycarbophil in einer Konzentration von ungefähr 0,5 bis ungefähr 1,5 Gew.-% vorliegt.

16. Zusammensetzung gemäß Anspruch 13, wobei der VEGF-Antagonist ein RNA-Aptamer ist, der so gestaltet ist, daß er VEGF antagonisiert.

17. Zusammensetzung gemäß Anspruch 13, wobei der VEGF-Antagonist ein neutralisierender Antikörper ist, der auf einen VEGF-Rezeptor gerichtet ist.

18. Zusammensetzung gemäß Anspruch 13, wobei die Zusammensetzung in der Lage ist, dem hinteren Abschnitt des Auges eine therapeutisch wirksame Menge des VEGF-Antagonisten bereitzustellen.

19. Zusammensetzung gemäß Anspruch 13, wobei die Zusammensetzung im Wesentlichen aus dem polymeren Suspensionsmittel und dem VEGF-Antagonisten besteht.

## Revendications

1. Utilisation d'un antagoniste de VEGF pour la préparation d'une composition ophtalmique topique destinée à être utilisée dans un procédé pour traiter ou prévenir des affections ophtalmiques du segment postérieur qui affectent le corps vitré, la rétine, le nerf optique ou la choroïde d'un mammifère nécessitant ce traitement, dans laquelle ladite composition comprend un agent de suspension polymérique et ledit antagoniste de VEGF, et dans laquelle une administration topique de ladite composition délivre une quantité thérapeutiquement efficace dudit antagoniste de VEGF auxdits corps vitré, rétine, nerf optique ou choroïde.

2. Utilisation selon la revendication 1, dans laquelle le mammifère est un humain.

3. Utilisation selon la revendication 1, dans laquelle l'agent de suspension polymérique comprend du polycarbophile.

4. Utilisation selon la revendication 3, dans laquelle le polycarbophile est présent à une concentration d'environ 0,5 à environ 1,5 pour cent en poids.

5. Utilisation selon la revendication 1, dans laquelle l'antagoniste de VEGF est un ARN aptamère conçu pour être un antagoniste de VEGF.

6. Utilisation selon la revendication 1, dans laquelle l'antagoniste de VEGF est un anticorps neutralisant qui cible un récepteur de VEGF.

7. Utilisation selon la revendication 1, dans laquelle l'antagoniste de VEGF est un peptide qui inhibe la liaison de VEGF à un récepteur de VEGF, ou un variant asymétrique de VEGF.

8. Utilisation selon la revendication 7, dans laquelle ledit antagoniste de VEGF est un peptide qui inhibe la liaison de VEGF à un récepteur de VEGF.

9. Utilisation selon la revendication 7 ou 8, dans laquelle ledit peptide est un peptide court contraint par une liaison disulfure.

10. Utilisation selon la revendication 9, dans laquelle ledit peptide a la séquence Ala-Thr-Trp-Leu-Pro-Pro-Arg (SEQ ID NO: 8).

11. Utilisation selon la revendication 1, dans laquelle ladite composition consiste essentiellement en un dit agent de suspension polymérique et ledit antagoniste de VEGF.

12. Utilisation d'un antagoniste de VEGF pour la préparation d'une composition ophtalmique topique destinée à être utilisée dans un procédé pour prévenir des affections ophtalmiques du segment postérieur qui affectent le corps vitré, la rétine, le nerf optique ou la choroïde d'un mammifère susceptible de développer de telles affections, dans laquelle ladite composition comprend un agent de suspension polymérique et ledit antagoniste de VEGF, et dans laquelle l'administration topique de ladite composition délivre une quantité thérapeutiquement efficace dudit antagoniste de VEGF auxdits corps vitré, rétine, nerf optique ou choroïde.

13. Composition ophtalmique topique destinée à être utilisée dans le traitement ou la prévention des affections ophtalmiques du segment postérieur qui affectent le corps vitré, la rétine, le nerf optique ou la choroïde d'un mammifère par administration topique à l'oeil, ladite composition comprenant une quantité thérapeutiquement efficace d'un antagoniste de VEGF et un agent de suspension polymérique.

14. Composition de la revendication 13, dans laquelle l'agent de suspension polymérique comprend du polycarbophile.

15. Composition de la revendication 14, dans laquelle le polycarbophile est présent à une concentration d'environ 0,5 à environ 1,5 pour cent en poids.

16. Composition de la revendication 13, dans laquelle l'antagoniste de VEGF est un ARN aptamère conçu pour être un antagoniste de VEGF.

17. Composition de la revendication 13, dans laquelle l'antagoniste de VEGF est un anticorps neutralisant qui cible un récepteur de VEGF.

18. Composition de la revendication 13, dans laquelle ladite composition est capable de délivrer une quantité thérapeutiquement efficace de l'antagoniste de VEGF au segment postérieur de l'oeil.

19. Composition selon la revendication 13, dans laquelle ladite composition consiste essentiellement en un dit agent de suspension polymérique et ledit antagoniste de VEGF.
